# EUROPEAN PATENT APPLICATION

(11) **EP 0 939 118 A1**
(43) Date of publication of application: **01.09.1999**
(21) Application number: 98102975.4
(22) Date of filing: 20.02.1998
(51) Int. Cl.: C12N 15/00

(54) **Method for isolating DNA and RNA from faeces**

(71) Applicant: Universiteit Maastricht, 6229 ER Maastricht (NL)
(72) Inventor: Thunnissen, Fredericus Bernardus Josephus M. Dr., 6200 MD MAASTRICHT (NL); Bang, Dang Duong c/o Universiteit Maastricht, 6200 MD MAASTRICHT (NL)
(74) Representative: Huygens, Arthur Victor

(57) **Abstract**

A method is provided for the isolation of DNA and RNA from a biological sample, in particular faeces, which comprises the step of rapidly mixing the sample with ice-cold water prior to adding any other liquid agent. A suitable amount of water relative to the amount of sample is about 50% v/v. Preferably, glass beads are added to promote mixing. Further steps include adding a detergent and a chelating agent to the mixture, such as SDS or Triton X-100 and EDTA, respectively, subjecting the mixture to a nucleic acid extraction with phenol/chloroform/isoamylalcohol, incubating, optionally chilling to form phenol needles, centrifuging and recovering the DNA and RNA from the water phase. The method is particularly useful for diagnostic purposes, for example the detection and diagnosis of human pathogens and mutated DNA in stool.

## Description

### Field of the Invention

The present invention is in the field of molecular biology and diagnostics. In particular, the invention relates to a new and efficient method for isolating and purifying DNA and RNA from biological samples and especially from faeces.

### Background of the Invention

A wide range of technologies involve the use of DNA and RNA in a variety of forms, in particular for experimental work and commercial production.

Advances in the area of diagnostics continue to utilize DNA and RNA in a variety of ways. For example, DNA probes are routinely used in the detection and diagnosis of human pathogens. Likewise, DNA and RNA is used in the detection of inborn or acquired genetic disorders.

There are numerous protocols for isolating and purifying DNA and RNA from biological samples. Also the simultaneous isolation of DNA and RNA has been described, often in conjunction with the isolation of proteins.

For example, Coombs et al., Anal Biochem. (1990) 188:338-343 (1990) disclose a method for simultaneously isolating DNA, RNA and proteins from the same biological sample which is essentially performed as follows: cell or tissue samples are homogenized in 4M guadinium. The homogenate is overlayered on a CsCl gradient. Following ultracentrifuging for 18 hours, the top guanidinium phase contains proteins, the upper layer of CsCl contains DNA, and RNA collects at the bottom of the ultracentrifuge tube. Purification of the DNA, RNA and protein fractions collected from the CsCl gradient is completed in the next 12-24 hours. The drawbacks of this method are that it takes 2-3 days to complete and it requires expensive equipment while only a limited number of samples can be processed simultaneously.

European patent application EP-A-0554034 (Chomczynski) discloses a method of simultaneously isolating DNA, RNA and proteins from biological samples wherein a solvent solution is applied which includes phenol and a guanidinium compound, preferably at a concentration of about 0.5-2M and at a pH of about 4-6. Utilizing this solvent solution and the method, isolation of substantially pure, substantially undegraded RNA may be completed in about one hour; similarly, the isolation of the DNA component and the proteins may take about 1-1.5 hours and about 1 hour, respectively.

U.S. Patent No. 5,405,951 (Woodart) discloses a method for purifying DNA from solution once obtained from its source which comprises the use of water soluble organic solvents such as ethanol, propanol, and isopropanol, eliminating the use of caustic and poisonous compositions such as chaotropes.

U.S. Patent No. 4,946,952 (Kiefer) discloses a method for extracting nucleic acids from a prokaryotic or eukaryotic cell or tissue extract by precipitation with a water soluble ketone, preferably after treatment with proteases.

U.S. Patent No. 4,921,952 (Longmire et al.) discloses a method for isolating DNA from eukaryotic cell and flow sorted chromosomes in which the organic extraction steps of conventional protocols are replaced with dialysis against polyethylene glycol. This dialysis removes proteolytic digestion products and detergent contaminants, while shear-degradation is reduced.

All methods described above suffer among other things from the drawback. that they are not generally applicable. For example, the methods do not work on the isolation of DNA, RNA and proteins from faeces, which may be caused by degradation and/or the presence of inhibitors. Isolation of DNA and RNA from faeces which is often desired for pathological purposes is still a major problem due to early degradation. No reliable isolation methods from faeces are available yet.

Thus, there is still a need for an efficient, accurate and more generally applicable method for isolating DNA, RNA and proteins from biological samples and in particular from faeces. The purpose of the present invention is to provide such a method.

### Summary of the Invention

According to one aspect of the invention a method is provided for the isolation of DNA and RNA from a biological sample and in particular faeces which comprises the step of rapidly mixing the sample with an ice-cold solubilizing agent prior to adding any other liquid agent. A suitable amount of solubilizing agent relative to the amount of sample is about 50% v/v. According to a preferred embodiment of the invention, glass beads are added to promote mixing.

The invention further comprises the steps of adding a detergent and a cheating agent to the mixture, subjecting the mixture to nucleic acid extraction with a solvent comprising phenol/chloroform, incubating, centrifuging and recovering the DNA and RNA from the water phase.

In a further preferred embodiment the mixture is rapidly chilled prior to the centrifuge step to form phenol needles.

The method is particularly useful for diagnostic purposes, for example the detection and diagnosis of human pathogens and mutated DNA in stool.

These and other aspects will be further described in the following specification.

### Brief description of the drawings

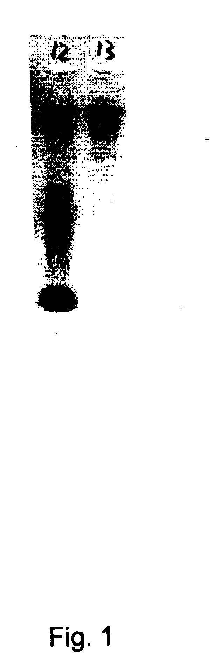

Figure 1 is an UV image of agarose gel with ethidium bromide staining of isolated nucleic acids from faeces sample with (13) and without (12) RNAse pretreatment.

### Detailed Description of the Invention

The term "isolated" as used in the present specification is meant to indicate that the particular component of interest, i.e. RNA or DNA, is separated or isolated from the other components to such a degree that there is no detectable contamination by those other components, as measured utilizing standard purity test procedures.

The term "undegraded" is meant to indicate that the component of interest is not degraded to any detectable degree, as measured by standard degradation test procedures, provided that degradation has not taken place before the start of the isolation procedure.

In accordance with the present invention an efficient and rapid isolation procedure for DNA and RNA is provided in which the extraction steps of conventional protocols are preceded by a step in which the biological sample is rapidly mixed with an ice-cold solubilizing agent and vortexed for about 30 to 60 seconds. The solubilizing agent is preferably water or PBS (phosphate buffered salt solution). The latter is suitably used at a pH in the range of about 7-7.5. Also suitable are other salt solutions in water, for example a 0.9% NaCl solution. While the physical-chemical mechanisms of this step are not yet fully clear, it is suggested that this surprising and simple step results in rehydration and possibly lysis of the cells. Care should be taken that this step is carried out rapidly (about 0.5-2 min.) and at low temperature, preferably at about 0°C, in order to avoid degradation. The amount of solubilizing agent relative to the solid is suitably about 50% v/v. In a preferred embodiment of the invention, glass beads are added to promote mixing.

The next steps of the isolation procedure are basically conducted in accordance with known techniques, depending on the specific nature of the sample to be treated. However, the specific combination of steps for the specific purposes is believed to be new.

In a typical example of the isolation of DNA and RNA from faeces, which is a preferred embodiment of the invention, the next step is the addition of a solvent solution comprising a detergent and a chelating agent, which solution is capable of dissolving mucus components which may be present in the sample and binding calcium and magnesium. A phenol/chloroform solution is then added to extract the nucleic acids present, the mixture is incubated on a water bath at about 65°C to inactivate remaining infectious components and RNAses, and centrifuged.

A suitable and preferred solvent solution comprises about 10% SDS as detergent and about 0.05-0.5M, preferably about 0.2M EDTA as chelating agent. Other detergents, such as Triton™ X-100, may be used as well. The solution to isolate nucleic acids is suitably a standard phenol/chloroform solution (suitably in a ratio of about 1:1) which may also comprise further components, such as an anti-foaming agent. A suitable anti-foaming agent is, for example, isoamylalcohol. A suitable ratio of the three components phenol/chloroform/isoamylalcohol is about 25:24:1. The incubation on the water bath usually takes about 10 minutes. The centrifuging time is also about 10 minutes at about 12,000 rpm and room temperature.

In a preferred embodiment of the invention the mixture is rapidly chilled prior to the centrifuge step in a dry ice-ethanol bath until solid phenol needles appear. The mixture is then centrifuged for about 10 minutes at about 12,000 rpm.

Although the isolation method according to the present invention is described above as a variety of steps, it is to be understood that all or at least a number of steps may be carried out simultaneously. This embodiment of the invention will be fully clear to the person skilled in the art and can be applied without inventive skill.

After centrifuging the upper aqueous phase is extracted once or twice with phenol/chloroform. Sodium acetate is then added to a final concentration of about 0.3M and the DNA is precipitated, for example by adding 2.5 volume of ethanol (96%) at -20°C for 20 minutes. The pellet is washed, suitably with 70% ethanol, dried, and resuspended in water at about 60°C.

The solution of nucleic acids can be stored at low temperature such that the RNA and DNA components are protected against degradation. A suitable temperature for storage is for example about -20°C.

The concentration and purity of the isolated nucleic acids can be determined by known methods, for example by the optical density (OD) method. The extent of purity can be determined by OD₂₆₀ measurements. According to the method of the present invention excellent purity values in a ratio of OD₂₆₀OD₂₈₀ > 1.8 are reached. The DNA isolated with the method of the present invention is also suitable for PCR technology, e.g. with K-ras primer, with or without dilution of DNA. For PCR application, the solution of nucleic acids is usually treated first with RNAse. Analysis of the DNA and RNA isolated according to the present invention occurs suitably according to methods known in the art.

The method according to the present invention is highly advantageous and distinct from other methods known in the art, since the isolation is very quick, carried out at low temperature of about 0°C, and can be used with up to about 50% w/v dry material. The method is particularly suitable for rapid DNA and RNA isolation from faeces samples (i.e. within about 30-45 minutes) and allows further molecular techniques for cancer screening, and diagnosis of infectious agents. However, the method can also be used for DNA and RNA isolation from other sources, for example urine, sputum, body liquors, tissue sections, microbial organisms (e.g. from soil samples), plants, etc., if appropriate with slight modifications which are evident to the persons skilled in the art. The method is also useful for the isolation of proteins from the biological samples, preferably conducted simultaneously with the isolation of DNA and RNA.

The following example illustrates the invention and is not intended as limiting the invention in any respect.

### Example 1

To 1 gram precooled stool (0°C) was added 1 ml precooled PBS pH 7 (0°C) and 1 gram of glass beads with a diameter of 1 mm. The mixture was vortexed for about 45 seconds.

Then 100 µl 10% SDS in 0.2 M EDTA (room temperature) was added and the mixture was vortexed for another 45 seconds. One ml of phenol/chloroform/ isoamylalcohol (25:24:1 v/v) was added and the mixture was incubated for 10 minutes on a water bath at 65°C.

Subsequently, the mixture was chilled in a dry ice/ethanol bath until the phenol was frozen, and centrifuged for 10 minutes, 12000 rpm at RT.

The upper aqueous phase was transferred to a fresh microcentrifuge tube and extracted twice with phenol/chloroform (1:1). Sodium acetate was then added.

The DNA was precipitated by adding 2.5 volume of pure alcohol (96%) at -20°C for 20 minutes. The precipitate was filtered off and washed twice with 70% ethanol. The remaining solid was dried and resuspended in 500 µl prewarmed (60°) water. The obtained solution with nucleic acids was stored at -20°C until further analysis.

## Claims

1. A method of isolating DNA and RNA from a biological sample, in particular faeces, which comprises the step of rapidly mixing the sample with a precooled solubilizing agent prior to adding any other liquid agent.

2. The method of claim 1, wherein the solubilizing agent is selected from the group of water and PBS.

3. The method of claim 1 or 2, further comprising the addition of glass beads.

4. The method of any of claims 1-3, wherein the amount of water relative to the amount of the sample is about 50% v/v.

5. The method of any of claims 1-4, wherein the mixture is vortexed for about 0.5-1 min.

6. The method of any one of claims 1-5, further comprising the steps of:
(a) adding a detergent and a chelating agent to the mixture;
(b) subjecting the mixture to a nucleic acid extracting solution;
(c) incubating the mixture at about 65°C;
(d) centrifuging the mixture; and
(e) recovering the DNA and RNA from the water phase.

7. The method of claim 6, wherein the detergent is selected from the group of SDS and Triton X-100 and the chelating agent is EDTA.

8. The method of claim 6, wherein the nucleic acid extraction solution comprises phenol, chloroform and optionally isoamylalcohol, preferably in a weight ratio of 25:24:1.

9. The method of claim 6, wherein the nucleic acid extraction solution comprises phenol, further comprising the step of chilling the mixture after the incubation step to obtain solid phenol needles.
